# EUROPEAN PATENT SPECIFICATION

(11) **EP 1 344 549 B1**
(45) Date of publication and mention of the grant of the patent: **04.11.2009**
(21) Application number: 03004721.1
(22) Date of filing: 04.03.2003
(51) Int. Cl.: A61M 25/00, B29C 47/24

(54) **Medical tubing and extrusion die for producing the same**
Medizinischer Schlauch und Extrusionsdüse zu seiner Herstellung
Tube médical et matrice d'extrusion permettant de le fabriquer

(30) Priority: 04.03.2002 JP 2002057098; 23.10.2002 JP 2002308011
(43) Date of publication of application: 17.09.2003
(73) Proprietor: TERUMO KABUSHIKI KAISHA, Tokyo (JP)
(72) Inventor: Kaneko, Takashi, c/o Terumo Kabushiki Kaisha, Ashigarakami-gun, Kanagawa (JP); Tsukamoto, Hideki, c/o Terumo Kabushiki Kaisha, Ashigarakami-gun, Kanagawa (JP); Matsuura, Shinichiro, c/o Mate Co., Ltd., Wake-gun, Okayama (JP); Akaiwa, Shuji, c/o Mate Co., Ltd., Wake-gun, Okayama (JP)
(74) Representative: Casalonga, Axel

(56) References cited:
- WO-A-00/01420
- US-A- 5 176 661
- US-A- 5 505 887
- US-A- 5 614 136
- US-A- 5 622 665
- US-A1- 2003 009 151

## Description

### BACKGROUND OF THE INVENTION

### Field of the Invention

The present invention relates to a medical tubing of excellent kink resistance which can be used as a sheath or catheter, for example. The invention relates in particular to an introducer sheath of excellent kink resistance in which such a medical tubing serves as the sheath portion and which is inserted into a body lumen and used for diagnostic or therapeutic purposes. This invention also relates to an extrusion die for manufacturing the medical tubing.

### Prior Art

Catheters are long, slender hollow tubular devices employed in modern medicine to carry out various forms of therapy. Such therapeutic methods include utilizing the long length of the catheter to administer medication directly to the affected region, using a catheter having at the distal end a balloon which when pressurized expands so as to dilate and open up a stenotic lesion within a blood vessel, and employing a catheter having at the distal end a cutter for resection at the site of a lesion to achieve patency. In addition to opening blood vessels, catheters are also used to deliver plugs to aneurysms, bleeding sites or nutrient vessels to close vascular flow channels. Catheters are commonly used as well for the insertion and placement within blood vessels of stents -- devices having a tubular shape with mesh-like sidewalls -- to maintain a state of patency at stenotic lesions within blood vessels.

One means of inserting a catheter into a body lumen is the Seldinger technique for accessing a blood vessel using an introducer sheath. A puncture needle such as an indwelling needle is inserted percutaneously into the vascular lumen, and a guide wire is passed through the needle from the proximal end thereof. The needle is then withdrawn, following which the sheath portion -- a slender hollow tubular body -- of the introducer sheath is inserted into the vascular lumen along the guide wire. Once the percutaneous opening has been enlarged by insertion of the sheath, the guide wire is withdrawn. The catheter is then inserted through the lumen of the sheath.

Characteristics required of the sheath portion of the introducer sheath used in this technique include resistance to kinking when inserted percutaneously into a body lumen, the ability to be inserted along bends in the body lumen, and small elongation set and moisture absorption. The sheath portions in prior-art introducer sheaths are made of materials such as nylon, polypropylene, fluoroplastics and polyurethane. However, such sheaths readily kink, are not adequately conducive to insertion, and lack sufficiently low elongation set and moisture absorption. Moreover, during insertion, the opening at the distal end of the sheath is pushed outward and enlarged, increasing resistance to insertion.

To improve resistance to kinking, a number of disclosures in the prior art rely on the use of a reinforcing body composed of metal coil or braid. Examples include providing a coiled body near the proximal end of the sheath (JP-A 61-71065), embedding a reinforcing body composed of a helically wound small-gauge wire within a layer of the hollow tubular sheath (JP-A 7-178181), and similarly embedding a coil (JP-A 5-192411 and JP-A 7-303703). In the art disclosed in JP-A 6-142212, instead of embedding a reinforcing body within the sheath, a metal coil composed of flat wire is placed at the interior of the sheath, which is a tubular body made of plastic.

However, all of these solutions entail complex production operations and high production costs. Moreover, embedding braided metal or coil may appear at first to improve kink resistance, but such reinforcement only increases the rigidity of the tubing itself and in fact does little to enhance the resistance to kinking. It is known that when the amount of bending exceeds a certain limit, this type of reinforcement loses its ability to conform to smaller radius of curvature changes, increasing rather than decreasing the tendency for the sheath to kink.

In light of the above, attempts have also been made to increase resistance to kinking by forming the sheath body as a multilayer tubing by coextrusion. One such example is the sheath made of two-layer tubing disclosed in JP-A 8-182765.

This prior-art sheath is claimed to have excellent kink resistance because a hard resin is used as the inner layer and a soft resin is used as the outer layer. Insertion and passage of the sheath are improved by exposing the inner layer made of hard resin at the tip portion thereof. However, the very fact that the inner layer made of hard resin is exposed at the distal portion of the sheath instead of being covered with soft resin suggests that the distal portion of the sheath has a poor kink resistance.

Other prior art hollow tubular bodies are disclosed in WO 00/01420 in which a filler-containing resin layer includes LCP fibers oriented helically and in US 5,622,665 disclosing a method for making a tubing having a differential stiffness for medical products such as a catheter.

### SUMMARY OF THE INVENTION

It is therefore one object of the invention to provide thin-walled medical tubing which is endowed with excellent maneuverability, including pushability, ability to transmit torque, trackability and kink resistance, and which can be used as a sheath or catheter. Another object of the invention is to provide an introducer sheath in which the sheath portion is made of such medical tubing. More specifically, one object of this invention is to provide medical tubing of improved kink resistance without enlarging the outside diameter or increasing the wall thickness, and another object is to provide an introducer sheath in which the sheath portion is made of this medical tubing. Another object of the invention is to provide an extrusion die for manufacturing the medical tubing.

Accordingly, in a first aspect, the invention provides a medical tubing comprising an elongated hollow tubular body which is open at both ends and extends continuously therebetween, which hollow tubular body has a filler-containing resin layer wherein the filler is oriented in a substantially circumferential direction with respect to the hollow tubular body. Preferably at least 30% of the filler in the resin layer is oriented in substantially the same direction.

In a preferred embodiment of the medical tubing of the invention, the hollow tubular body is composed of at least two resin layers and has a filler-free resin layer on at least the outside and inside of the filler-containing resin layer. The filler-containing resin layer in this embodiment is typically composed of a principal resin, an adhesive resin and a filler. The filler is preferably composed of whiskers.

In a second aspect, the invention provides an introducer sheath composed of a sheath portion which is an elongated hollow tubular body having a distal end and a proximal end and which has a lumen, and composed also of a hub which is a housing having a central cavity and which is disposed at the proximal end of the sheath portion so that the central cavity communicates with the sheath lumen. The sheath portion has a filler-containing resin layer, which filler is oriented within the resin layer in a substantially circumferential direction with respect to the hollow tubular body.

In a preferred embodiment of the introducer sheath of the invention, the sheath portion has a straight section of substantially uniform diameter and a tapered section which is provided distal to the straight section and decreases in diameter toward the distal end of the sheath portion.

In a third aspect, the invention provides an extrusion die for producing medical tubing, which die includes a die body having at least two resin inlets, a flow-combining section located at an extrusion downstream end of the die body and, at the interior thereof, branched channels that communicate independently between each resin inlet and the flow-combining section and meet at the flow-combining section; a mandrel which is a cylindrical body situated at the interior of and passing through the die body, has a downstream end in the extrusion direction that extends beyond the flow-combining section, and has a cylindrical outer peripheral surface that contacts the resin passing through the die; and a female diet member which is disposed coaxially with the mandrel at the downstream end of the die body in the extrusion direction and has an inner peripheral surface. The outer peripheral surface of the mandrel adjoins the inner peripheral surface of the female die member across an intervening gap of annular cross-section. The gap communicates with the flow-combining section and forms a resin flow channel within the female die member. The mandrel, the female die member or the both are axially rotatable relative to the direction of extrusion.

In a preferred embodiment of the extrusion die of the invention, the resin flow channel within the female die member has a tapered section where the outer peripheral surface of the mandrel, the inner peripheral surface of the female die member or the both gradually decrease in diameter in the downstream direction from the flow-combining section of the die body and the flow channel gradually diminishes in cross-sectional area, and an extrusion section which is situated downstream from the tapered section.

These and other objects, features and advantages of the invention will become more apparent upon consideration of the following detailed description and the accompanying figures.

### BRIEF DESCRIPTION OF THE DRAWINGS

FIG. 1 is a perspective view showing the basic construction of the medical tubing according to the invention.
FIG. 2 is a similar partially cutaway view of a medical tubing according to the present invention, but showing the filler in the resin layer oriented in the circumferential direction of the tubing.
FIG. 3 is a cross-sectional view of a medical tubing of laminated construction according to the invention, taken perpendicular to the tubing axis.
FIG. 4 is a perspective view of an embodiment of an introducer sheath.
FIG. 5 is a substantially cross-sectional view of an embodiment of a laminated tubular body coextrusion die.
FIG. 6 is a schematic view showing an example of a laminated tubular body extrusion line containing the extrusion die.
FIG. 7 is a partially cross-sectional view showing an example of a direct coupling arrangement between the extrusion die and a drive unit.
FIG. 8 shows schematic views of offset coupling arrangements between the extrusion die and a rotational die drive unit, using a belt pulley (FIG. 8A) or gears (FIG. 8B).
FIG. 9 shows a cross-sectional view of the resin lamination step at the interior of the extrusion die, and a cross-sectional view of the tubing following lamination.
FIGS. 10A and 10B are cross-sectional views illustrating the effect of rotation.

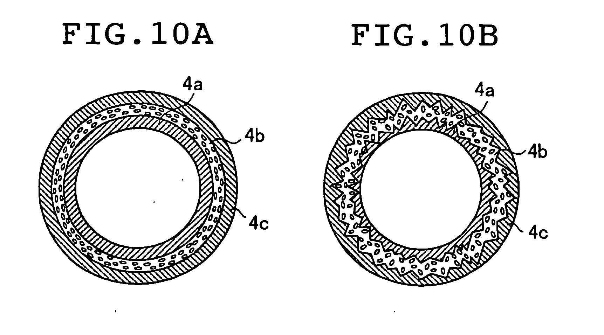

FIG. 10A shows a laminated tubing according to the invention, and FIG. 10B shows a defective tubing according to a comparative example.

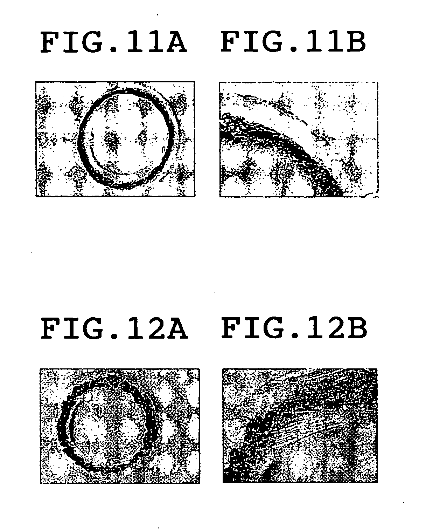

FIGS. 11A and B show the effect of rotation . These are cross-sectional images of a tubing produced by rotation at 100 rpm. The images were taken through an optical microscope, input to a computer and printed out. FIG. 11A was obtained at a magnification of 50X, and FIG. 11B was obtained at a magnification of 200X.
FIGS. 12A and B are cross-sectional images of a tubing produced without rotation in a comparative example. The images were taken through an optical microscope, input to a computer and printed out. FIG. 12A was obtained at a magnification of 50X, and FIG. 12B was obtained at a magnification of 200X.

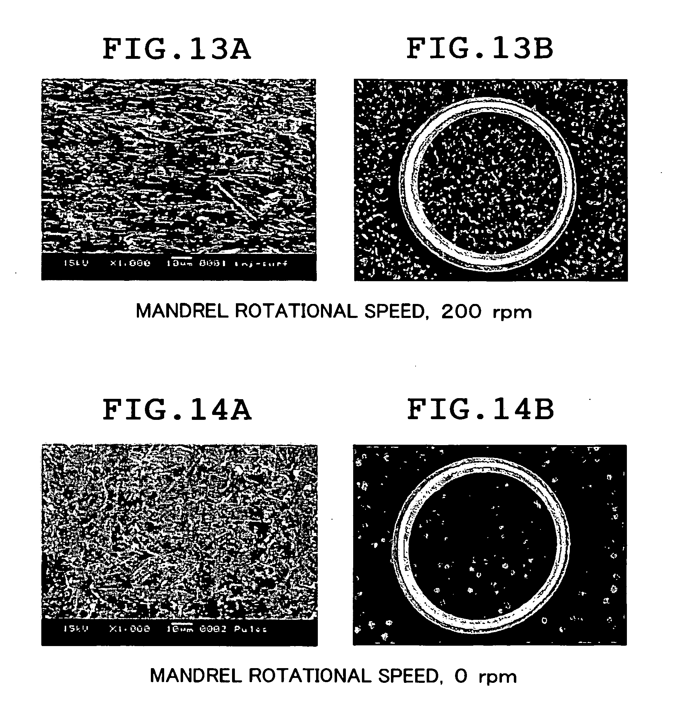

FIG. 13A is a scanning electron micrograph (magnification, 1,000x) showing an enlarged portion of a cross-section of the medical tubing obtained in Example 2 according to the invention (mandrel rotational speed, 200 rpm), and FIG. 13B is a cross-sectional micrograph of the medical tubing obtained in Example 2 (magnification, 50x).
FIG. 14A is a scanning electron micrograph (magnification, 1,000x) showing an enlarged portion of a cross-section of the medical tubing obtained in Comparative Example 2 (mandrel rotational speed, 0 rpm), and FIG. 14B is a cross-sectional micrograph of the medical tubing obtained in Comparative Example 2 (magnification, 50x).

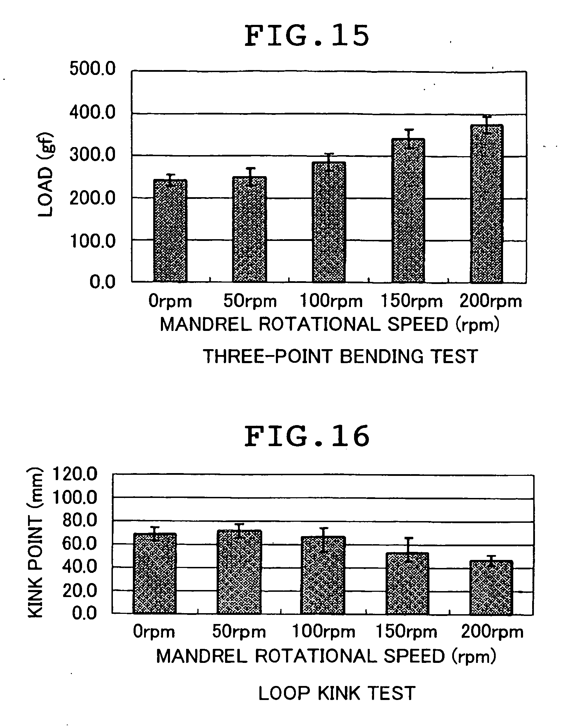

FIG. 15 is a graph of the results from three-point bending tests on the medical tubings obtained in Examples 2 to 5 of the invention and in Comparative Example 2. The graph shows the relationship between the rotational speed of the mandrel during production and the three-point bending load.
FIG. 16 is a graph of the results of loop kink tests on the medical tubings obtained in Examples 2 to 5 and in Comparative Example 2. The graph shows the relationship between the rotational speed of the mandrel during production and the kink point.

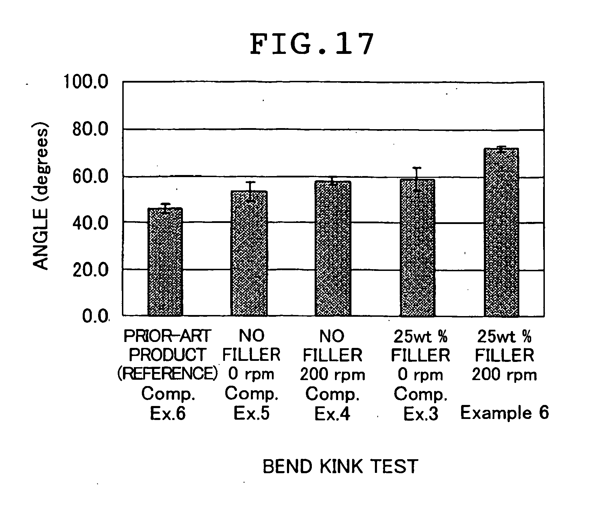

FIG. 17 is a graph of the results of bend kink tests on the sheaths obtained in Example 6 of the invention and in Comparative Examples 3 to 6. The graph shows the bending angle of the sheath when kinking occurred.

### DETAILED DESCRIPTION OF THE INVENTION

The medical tubing, introducer sheath and extrusion die are described below while referring to the accompanying figures. The figures show some embodiments of the medical tubing, introducer sheath and extrusion die for the purpose of illustrating the invention, although the medical tubing, introducer sheath and extrusion die of the invention are not limited to the embodiments shown in the figures.

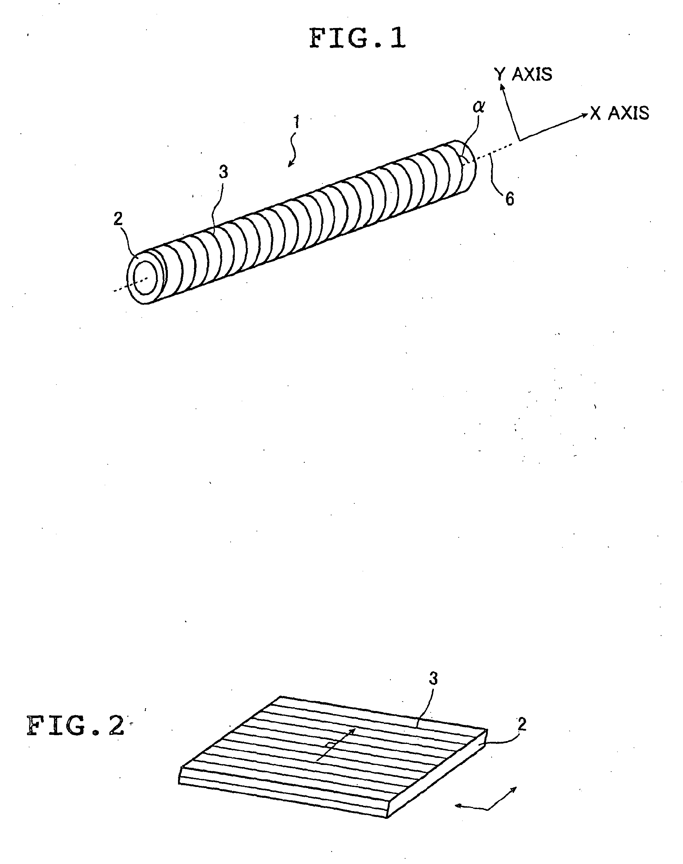

FIG. 1 is a perspective view showing the basic construction of the medical tubing according to the invention. As shown in FIG. 1, the inventive medical tubing 1 has an elongated hollow tubular body which is open at both ends and extends continuously therebetween. This medical tubing 1 has a filler-containing resin layer 2. The particular medical tubing 1 shown in FIG. 1 is a single-layer hollow tubular body formed only of a resin layer 2 that contains a filler 3.

The filler 3 within the resin layer 2 is oriented in a substantially circumferential direction with respect to the hollow tubular body. In the figure, the oriented state of the filler 3 is shown schematically as broad tape-like lines for easy visualization. However, as is shown later in photographs taken with a scanning electron microscope (SEM), individual particles of filler are oriented over the entire body within a certain angular range with respect to the longitudinal axis. Fine lines having such an orientation are observable upon examination with a scanning electron microscope.

In the medical tubing 1 shown in FIG. 1, letting the direction of the axis 6 of the tubing 1 (the hollow tubular body) be the X axis and the circumferential direction be the Y axis, the filler 3 is oriented at an angle α to the X axis. FIG. 2 illustrates the oriented state of the filler 3 in the resin layer 2 using partially cutaway views in which a portion of the sidewall of a tubing (hollow tubular body) has been cut and the tubing opened up and laid flat. FIG. 2 shows a state in which the filler 3 is oriented at an angle of 90 degrees to the tubing axis. In the latter case, the direction of orientation by the filler 3 coincides with the circumferential direction of the tubing.

Because the filler 3 present in the resin layer 2 is oriented in a substantially circumferential direction with respect to the tubular body, i.e., the medical tubing 1 of the invention has improved resistance to kinking when bent laterally; that is, when bend in a direction perpendicular to the axial direction of the tubing.

The inventive medical tubing 1 is capable of reversible deformation with respect to localized bending, and thus has excellent kink resistance.

The filler 3 need not all be uniformly oriented in the same direction. That is, the resin layer 2 may have present therein filler 3 which is oriented in mutually differing directions. In such a case, it is preferable for at least 30% of the filler 3 present within the resin layer 2 to be oriented in mutually the same direction. It is more preferable for at least 40%, and most preferable for at least 50%, of the filler 3 to be oriented in mutually the same direction.

FIG. 1 shows a case in which the medical tubing 1 according to the present invention is composed solely of a single resin layer 2 containing filler 3, but the medical tubing of the invention is not limited to the embodiment shown in this diagram. The medical tubing of the invention is a hollow tubular body having a plurality of laminated resin layers, such as three resin layers. Such a multilayer medical tubing has a construction in which a filler-free resin layer is provided on at least the outside and inside of a filler-containing resin layer 2. It is especially preferable for such a multilayer medical tubing to be a hollow tubular body having a three-layer construction in which filler-free resin layers are provided on both the outside and inside of a filler-containing resin layer.

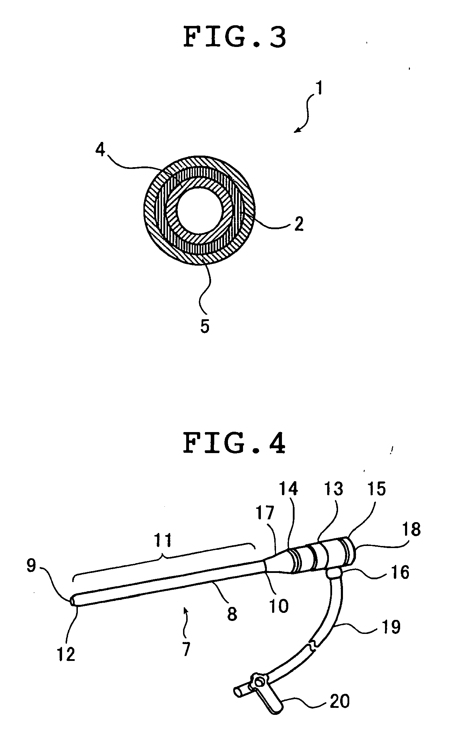

FIG. 3 is a cross-sectional view taken perpendicular to the axis of a medical tubing according to the invention which has such a three-layer construction. The medical tubing 1 shown in FIG. 3 is composed of a filler-containing resin layer 2 provided on the outside and inside thereof with filler-free resin layers (inside layer 4 and outside layer 5). This three-layer construction in which the filler-free resin layers 4 and 5 cover the filler-containing resin layer 2 on the inside and outside thereof provides good safety for patients because the filler is not exposed on the inner and outer surfaces of the tubing.

In the medical tubing 1 of the invention, the resin making up the filler-containing resin layer 2 and the filler-free resin layers 4 and 5 which may be present on the inside and outside thereof is generally a thermoplastic resin (i.e., plastic) or a thermoset or thermally crosslinkable resin such as rubber. Specific examples include various types of thermoplastic resins and polymer derivatives thereof, among them polyesters such as polyethylene terephthalate and polybutylene terephthalate, polyester elastomers containing such polyesters as the hard segments, polyolefins such as polyethylene and polypropylene, polyolefin elastomers, copolyolefins prepared with metallocene catalysts, vinyl polymers such as polyvinyl chloride, polyvinylidene chloride (PVDC) and polyvinylidene fluoride (PVDF), polyamides such as nylons, nylon-containing polyamide elastomers (PAE), polyimides, polystyrenes, styrene-ethylene-butylene-styrene block copolymers (SEBS), polyurethanes, polyurethane elastomers, ABS resins, acrylic resins, polyarylates, polycarbonates, polyoxymethylene (POM), polyvinyl alcohol (PVA), fluoroplastics (ETFE, PFA, PTFE), ethylene-vinyl acetate saponification products, ethylene-vinyl alcohol copolymers, ethylene-vinyl acetate, cellulose plastics such as carboxymethylcellulose, methyl cellulose and cellulose acetate, vinyl polysulfones, liquid crystal polymers (LCP), polyether sulfones (PES), polyetheretherketones (PEEK), polyphenylene oxides (PPO) and polyphenylene sulfides (PPS). Additional examples include thermoset or thermally crosslinkable resins such as vulcanized rubbers, silicone resins, epoxy resins and two-part reactive polyurethane resins.

Polymer alloys containing any of the foregoing thermoplastic resins and thermoset or thermally crosslinkable resins may also be used. Of the above, polyester elastomers, polyamide elastomers and polyurethane elastomers are preferred because they have excellent shape recoverability and are thus capable of conferring the medical tubing with good resistance to kinking.

When the medical tubing is a hollow tubular body having a multilayer construction like that shown in FIG. 3, it is preferable for the filler-containing resin layer 2 to be composed of the same type of resin as that making up the filler-free resin layers 4 and 5 on the inside and outside thereof. By using the same type of resin in the filler-containing resin layer 2 as in the filler-free resin layers 4 and 5, excellent interlaminar adhesion can be achieved.

To further improve the interlaminar adhesion, it is advantageous for the filler-containing resin layer 2 to include as well, in addition to the principal resin of which the filler-free resin layers 4 and 5 also are composed, another resin, such as an adhesive resin. "Adhesive resin," as used herein, refers broadly to resins having adhesive properties with respect to the resin making up the filler-free resin layers 4 and 5. Hence, it is suitably selected in accordance with the type of resin making up the filler-free resin layers 4 and 5. For instance, in an example according to the invention that is described later in this specification, a polyester elastomer resin is used in the filler-free inner and outer layers. Together with the same polyester elastomer resin, the filler-containing intermediate layer includes, as the adhesive resin, an ethylene-ethyl acrylate copolymer (EEA) having adhesive properties with respect to the polyester elastomer resin.

In cases where the medical tubing of the invention is manufactured by coextrusion using the subsequently described extrusion die of the invention, the adhesive resin can also be used as a melt viscosity regulator between the filler-containing intermediate layer and the filler-free inner and outer layers.

When the filler-containing resin layer 2 contains an adhesive resin, the amount of adhesive resin, based on the overall weight of the materials (resin, adhesive resin, filler) making up the resin layer 2 is preferably 5 to 50 wt %, and more preferably 10 to 30 wt %. A proportion of adhesive resin in the filler-containing resin layer 2 within this range provides the medical tubing produced therefrom with outstanding mechanical strength and imparts the filler-containing resin layer 2 with excellent adhesion to the filler-free resin layers 4 and 5 on the inside and outside thereof.

When the filler included in resin layer 2 is a filler that is widely used in resins and has a high aspect ratio, particularly one that is flat, it is easy to orient. Illustrative examples of such fillers include potassium titanate, wollastonite, sonolite, gypsum fibers, aramid fibers, aluminum borate, carbon fibers, glass fibers, talc, mica, glass flakes, and polyoxybenzoyl whiskers. The filler may be of shape that is, for example, fibrous, acicular, tabular or granular. Illustrative examples of commercial products suitable as the filler include whiskers and chopped fibers. Of these, potassium titanium whiskers are especially preferred because they are readily available and ease to handle.

Alternatively, the filler included in resin layer 2 may be a flat powder of a soft magnetic metal, specific examples of which include carbonyl iron and iron-based alloys such as iron-silicon alloys, iron-nickel alloys, iron-cobalt alloys, iron-aluminum-silicon alloys, iron-chromium alloys, iron-chromium-silicon alloys and iron-copper-niobium-silicon-boron alloys. When this type of soft magnetic metal flat powder is oriented in the circumferential direction, numerous filler particles having electromagnetic shielding properties become present in an overlapping arrangement (sometimes achieving an effect like that of fallen leaves), making it possible to fully elicit the electromagnetic shielding effects of the flat powder of soft magnetic metal.

It is generally preferable for the resin layer 2 to contain 10 to 80 parts by weight of filler per 100 parts by weight of the overall material (resin, adhesive resin, and filler) making up the resin layer 2. In the case of single-layer medical tubing which, as shown in FIG. 1, is composed of only a filler-containing resin layer 2, the amount of filler 3 is preferably 10 to 50 parts by weight, and most preferably 15 to 30 parts by weight, per 100 parts by weight of the total weight of the materials making up the resin layer 2. In the case of medical tubing having a multilayer construction like that shown in FIG. 3, the amount of filler 3 is preferably 15 to 80 parts by weight, and most preferably 20 to 60 parts by weight, per 100 parts by weight of the total weight of the materials making up the resin layer 2. An amount of filler in the resin layer that falls within the above range provides the medical tubing with excellent maneuverability, including pushability, ability to transmit torque, trackability and kink resistance. A filler content within the above range is also highly desirable for holding down the production costs of the medical tubing.

FIG. 4 is a perspective view of an example of an introducer sheath in which the medical tubing of the invention is used as the sheath portion. The inventive medical tubing has an excellent resistance to kinking, and is thus highly suitable for use as the sheath portion of an introducer sheath. The introducer sheath 7 of the invention shown in FIG. 4 has a sheath portion 8 which is an elongated hollow tubular body having a distal end 9 and a proximal end 10 and which has a lumen. The introducer sheath 7 also has a hub 13 which is a housing having a central cavity and which is connected to the proximal end 10 of the sheath portion 8 so that the central cavity of the housing communicates with the hollow tubular body lumen of the sheath portion 8. The sheath portion 8 has a filler-containing resin layer. The filler in the resin layer is oriented in a substantially circumferential direction with respect to the longitudinal axis of the sheath portion 8. This sheath portion 8 is preferably a medical tubing according to the present invention as described above.

The sheath portion 8 has a straight section 11 of substantially uniform diameter, and a tapered section 12 which is provided on the distal side of the straight section 11 and decreases in diameter toward the distal end 9 of the sheath portion. By decreasing in diameter (tapering) toward the distal end 9 of the sheath portion 8, the tapered section 12 improves maneuverability when the sheath portion 8 is introduced into a body lumen.

In FIG. 4, a distal end 14 of the hub 13 having a central cavity is typically inserted into an opening at the proximal end 10 of the sheath portion 8, thereby connecting the distal end 14 of the hub 13 with the proximal end 10 of the sheath 8 so that the central cavity of the hub 13 communicates with the lumen of the sheath portion 8. However, the connection between the distal end 14 of the hub 13 and the proximal end 10 of the sheath portion 8 is not limited to the arrangement just described. Another acceptable arrangement is one in which the proximal end 10 of the sheath portion 8 is inserted into an opening in the distal end 14 of the hub 13, thereby connecting the distal end 14 of the hub 13 with the proximal end 10 of the sheath portion 8 so that the central cavity of the hub 13 communicates with the lumen of the sheath portion 8.

The hub 13 may be made of any of the synthetic resins listed above as examples of the resin used in the medical tubing of the invention. Synthetic resins such as polyethylene resins, polypropylene resins, polyamide resins, polycarbonate resins and polystyrene resins are preferred as the synthetic resin used to form the hub for reasons having to do with biocompatibility, mechanical strength, melt processability and production costs.

The introducer sheath 7 of the invention may include also other elements in the arrangement for connecting the proximal end 10 of the sheath portion 8 with the distal end 14 of the hub 13. For example, in FIG. 4, a support 17 having a tapered construction is provided between the distal end 14 of the hub 13 and the proximal end 10 of the sheath portion 8. The support 17 may be formed as an extension of the distal end of the hub 13 or may constructed as a separate member from the hub 13. By providing this support 17, kinking of the sheath portion 8 can be prevented. To prevent the sheath portion 8 from kinking, materials preferred for use in the support 17 include styrene, olefin, and polyester-based thermoplastic elastomers having flexibility.

The introducer sheath 7 shown in FIG. 4 is provided, at a proximal end 15 of the hub 13, with a valve 18 to prevent the reflux of blood and other fluids. No particular limitation is imposed on the material of which the valve 18 is made, although the valve 18 is preferably composed of a material such as silicone, latex, butyl rubber or isoprene rubber. This valve 18 most often has a construction in which a cylindrical body of substantial thickness has a slit in each of the two bases of the cylinder such that the two slits are arranged as cross-slits which intersect only at the interior of the body. However, the valve construction is not subject to any particular limitation insofar as the reflux of blood and other fluids can be prevented. For example, valves having multiple overlapping Y slits, cross-slit and slit; and duckbill valves, and other known valves may be used.

The hub 13 has formed in a sidewall thereof a branching area 16 which communicates between the central cavity within the hub 13 and the exterior. The branching area 16 has connected thereto one end of a branch tube 19 which is a hollow tubular body. The branch tube 19 has attached thereto, at another end not connected to the branching area 16, a three-way stopcock 20.

It should be noted, however, that the introducer sheath 7 of the invention need only have a sheath portion 8 and a hub 13. The other elements shown in FIG. 4 are not always essential, and may be substituted with elements having similar functions but different shapes or constructions.

The sheath portion 8 may include, in addition to the above-described construction of the inventive medical tubing, other elements helpful to the use of the introducer sheath. A preferred example of such an additional element is the presence, in some or all parts of the sheath portion 8, of a radiopaque material such as bismuth oxide, tungsten or barium sulfate for verifying the position of the sheath portion 8 within the blood vessel into which it has been inserted. Within the sheath portion 8, such a radiopaque material may be included in the filler-containing resin layer 2 or in filler-free resin layer 4 or 5 provided on the inside or outside of the filler-containing resin layer 2.

Moreover, the inner surface or outer surface of the sheath portion 8 may be coated with a thin film of an antithrombogenic resin to provide an introducer sheath having even better biocompatibility. The antithrombogenic resin coated onto the inner surface or outer surface of the sheath portion 8 may be any one or combination of various suitable resins known to the art. Preferred examples of such resins include poly-2-methoxyethyl acrylate (PMEA), polyhydroxyethyl methacrylate, and copolymers of hydroxyethyl methacrylate (HEMA) and styrene (St) such as HEMA-St-HEMA block copolymers.

It is also possible to coat the inner or outer surface of the sheath portion 8 with a thin film of a resin that is slippery when wet. This makes it possible to obtain an introducer sheath having even better insertability into body lumens. The slippery resin coated onto the inner or outer surface of the sheath portion 8 may be any one or combination of various suitable resins known to the art. Preferred examples include polyvinyl pyrrolidone (PVP), polyethylene glycol (PEG), polydimethyl methacrylate (PDMAA), polysaccharides (e.g., hyaluronic acid) and poly(methyl vinyl ether-maleic anhydride) copolymers.

The outside diameter of the sheath portion 8 is not subject to any particular limitation, although a diameter of about 0.8 to 11 mm is generally preferred, and a diameter of about 1.0 to 5.0 mm is especially preferred. The inside diameter of the sheath portion 8 is also not subject to any particular limitation, but is preferably about 0.7 to 10 mm, and most preferably about 0.9 to 4.7 mm. The wall thickness of the sheath portion 8, while also not subject to any particular limitation, is generally about 0.1 to 0.3 mm, and preferably about 0.1 to 0.25 mm. The introducer sheath 7 has a length which is likewise not subject to any particular limitation, but is preferably about 10 to 300 mm, and most preferably about 50 to 150 mm.

The medical tubing of the invention may be manufactured by any process capable of producing a hollow tubular body having a resin layer containing filler oriented in an circumferential direction with respect to the axial direction of the tubular body. The process may involve, for example, producing a sheet of resin containing a filler oriented circumferentially to the axial direction of the tubing to be manufactured; rolling the sheet up into a tube, either by itself or after lamination with a sheet made of filler-free resin; and joining together the butted edges of the tube. Another suitable process is to helically coil a ribbon of resin containing a filler oriented in the lengthwise direction thereof onto the surface of a tubular body made of resin so as to provide a resin layer containing filler oriented circumferentially to the axial direction of the tubular body.

Alternatively, a single-layer medical tubing composed of a resin layer containing filler oriented circumferentially to the axial direction thereof may be produced using an extruder which rolls the filler-containing resin material while molten in the circumferential direction and extrudes the melt in the axial direction.

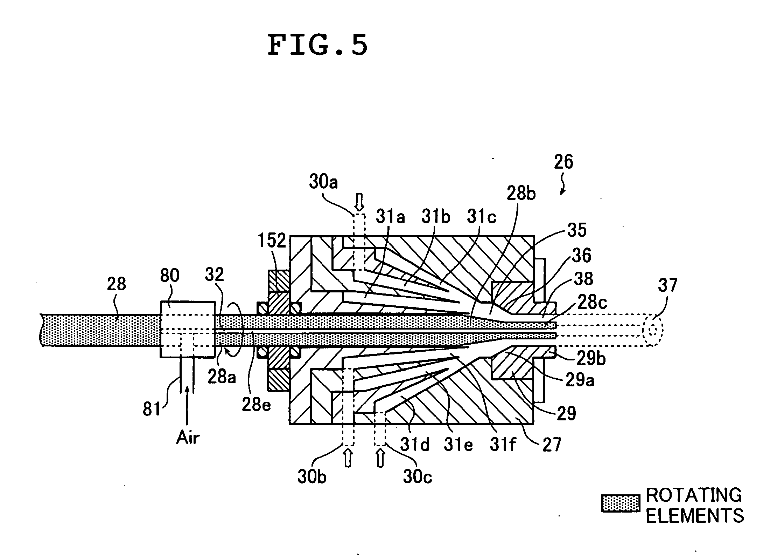

Medical tubing having a multilayer structure, exemplified by the medical tubing with a three-layer structure shown in FIG. 3, can be produced by coextruding a plurality of resin layers using an extruder equipped with a die for this purpose, one example of which is the extrusion die 26 of the invention shown in FIG. 5. The procedure for manufacturing the three-layer medical tubing shown in FIG. 3 using the extrusion die shown in FIG. 5 is described in detail below. In the description that follows, the left side of the figure is the upstream side in the extrusion direction. The right side of the figure, which is the side where the tubing 37 is extruded, is the downstream side.

The extrusion die 26 shown in FIG. 5 has a die body 27, a mandrel 28 which is situated at the interior of and passes through the die body 27, and a female die member 29 which is disposed coaxially with the mandrel 28 at the downstream end of the die body 27 in the extrusion direction. The mandrel 28,'the female die body 29 or the both are axially rotatable relative to the direction of extrusion. Either the mandrel 28, the die 29 or the both may be rotated in the present invention, although the description that follows relates primarily to an embodiment in which only the mandrel 28 is rotated.

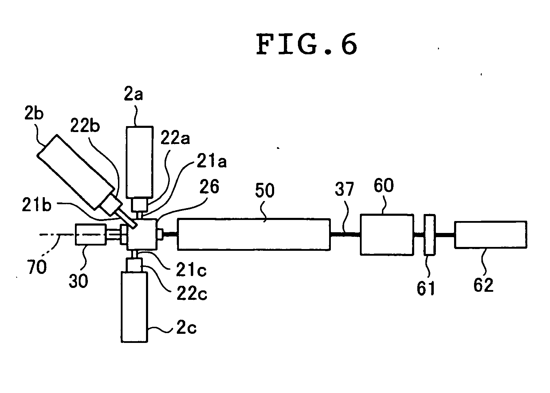

The mandrel 28 shown in FIG. 5 has a hollow construction with a central cavity 28e that extends in the axial direction. The mandrel 28 is composed of, from the upstream side, a mandrel shaft 28a connected to a drive unit 30 (FIG. 6) for rotating the mandrel 28, a tapered section 28b, and a rotating point 28c.

The resin flow channel provided in the die body 27 has, from the upstream side, resin inlets 30a to 30c; branch channels 31a to 31c which, after opening up into tubular passages from the respective resin inlets 30a to 30c, gradually decrease in diameter and meet at a flow-combining section 35. The branch channels 31a to 31c each communicate independently with the flow-combining section 35. Downstream from the flow-combining section 35, there is formed a female die member/mandrel point resin flow-combining channel (also referred to hereinafter as the "extrusion flow channel") 38 where the molten resin is extruded while being subjected to rotation in the circumferential direction. The extrusion flow channel 38 has a diameter which is substantially the same as the inside diameter of the subsequently described the die land 29b of the female die member 29, and is of a dimension and shape close to the outside diameter of the extruded tubing 37.

The die body 27 has overall a manifold construction composed of a resin flow channel enclosed within a tubular housing. It may be built by, for example,assembling a plurality of die members.

The female die member 29 is positioned at the most downstream side of the resin flow channel in the die body 27. The female die member 29 has at the interior thereof a cylindrical space defined by an inner peripheral surface of larger diameter than the outer peripheral surface of the mandrel 28. At least the upstream end (in the extrusion direction) of the cylindrical space communicates with the flow-combining section 35 of the resin flow channel within the die body 27. The female die member 29 has, from the upstream side, a die stepper 29a and a die land 29b which is positioned at the most downstream end of the resin outlet. The female die member 29 is fastened to the die body 27 with a die holder.

The mandrel shaft 28a is held at the upstream end of the die body 27 by a shaft seal 152 which prevents resin leakage. The mandrel shaft 28a does not come into contact with the resin stream, but the tapered section 28b of the mandrel and the rotating point 28c of the mandrel do come into contact with the resin stream.

The embodiment described above is a preferred embodiment in which the resin flow channel, the mandrel 28, and the female die member 29 form a continuous arrangement wherein each of these components has a linearly tapered structure. Alternatively, instead of having a linearly tapered construction, each of these components may narrow to a smaller diameter in some other desired shape defined by smooth curves.

Next, a method of forming a laminated tubular body using the foregoing extrusion die 26 is described. In the figures referred to below, symbols which are the same as those in FIG. 5 or in other diagrams represent like or corresponding features. Repeated explanations of like features are omitted.

FIG. 6 is a schematic view of an extrusion line for producing a laminated tubular body using the above-described rotating point-type extrusion die 26 The figure illustrates how three-layer medical tubing composed primarily of a thermoplastic polymeric material is produced.

Commercially available equipment may be used for each piece of equipment shown in FIG. 6 other than the extrusion die 26. If necessary, the extruder cylinder, screw and other components may be made of special metallic materials or substances such as corrosion-resistant materials and wear-resistant materials. Extruder specifications such as size and plasticating capacity may be selected as appropriate for the intended use of the inventive medical tubing.

FIG. 6 shows an arrangement in which three extruders 2a, 2b and 2c are used to produce a three-layer tubular body. For the sake of illustration, in a case where three layers are to be coextruded from two types of polymer to form a three-layer laminated tubular body, the use of three extruders allows each of the three layers to be formed of material from a different extruder. However, in a case where the inner and outer layers are made of the same material, by using two extruders, the material for the inner and outer layers can be supplied from one of the extruders and the material for the intermediate layer can be supplied from the other extruder.

Even when the inner and outer layers are made of the same type of polymer, using three extruders and feeding the material for each layer from a different extruder is advantageous because the polymer can be distributed in the desired amounts and the inner and outer layers can be easily adjusted to the respective desired thicknesses.

The respective materials within extruders 2a to 2c are delivered under pressure through respective adapters 21a to 21c and resin inlets 30a to 30c into the extrusion die 26. Gear pumps 22a, 22b and 22c may or may not be used, although their use is preferred when the product is required to have dimensional precision.

After delivery to the extrusion die 26, the resin is shaped therein into a laminated tubular body and continuously extruded from the extrusion die 26. The tubing 37 extruded from the die land 29b is solidified in a coagulation bath 50, then continuously hauled off with a haul-off unit 60. Next, the tubing is measured with a laser outside diameter measuring system 61, following which it is collected by winding with a windup unit 62 or is cut with a cutter.

The type of coagulation bath 50 will differ depending on whether the material used to form the extruded tubing is a thermoplastic resin, a resin dope in a solvent, or a thermoset resin. For example, the bath may be one that solidifies the extrudate by means of cooling, chemicals or heating.

If extrusion is carried out using a thermoplastic resin, solidification is generally carried out by cooling in a water bath. Although not always necessary, when a water bath is employed, to improve the roundness of the tubular body, use may also be made of auxiliary equipment such as a resin low-pressure sizing unit or a vacuum water tank. Of these, the use of a low-pressure sizing unit is preferred.

During extrusion of the tubing, use may be made of a core material 70 such as a solid core material (e.g., copper wire) or a liquid or gas.

Using a solid core material 70 makes it possible to easily maintain the inside diameter of the soft, readily deformable resin that has just been extruded from and shaped by the die land 29a. Gases such as air or nitrogen are often used as the core material because of their low cost and to eliminate the need for withdrawing a metal core material. The core material used in the example shown in FIG. 5 is a gas, and specifically air.

The method of feeding the core material, the drive unit 30 coupling arrangement, and the method of transmitting rotation from the drive unit 30 to the mandrel 28 may be suitably selected according to the type of core material. The core material feeding method is also a factor in selection of the drive unit 30 coupling mechanism and the manner in which rotation is transmitted to the mandrel 28. When air is used as the core material, air coupling is used. Examples of methods that may be used to transmit rotation include direct coupling and offset coupling.

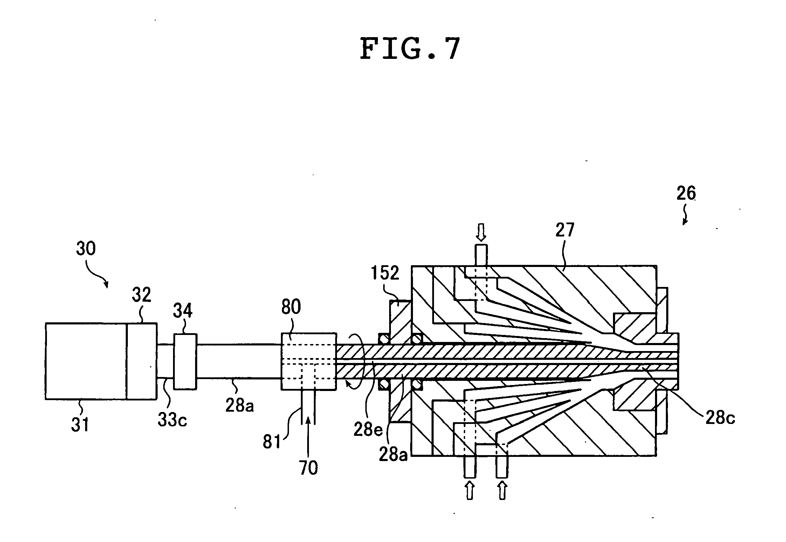

FIG. 7 is a partially cross-sectional view, showing an example of a direct coupling arrangement that can be employed between the extrusion die 26, the drive unit 30 and the core material feeding means in cases where air or a liquid is used as the core material 70.

In this example, the drive unit 30 employs an ES servomotor 31. ES servomotors 31 are preferred for good rotational precision and control of the rotating point 28c portion of the mandrel 28, and are commercially available. The ES servomotor 31 generally has speed reducer 32 mounted thereon for adjustment of the rotational speed at which feed is to be carried out. Based on the motor performance and the rotational speed to be controlled, a combination of speed reducer 32 elements may be suitably selected to impart a speed reduction ratio in keeping with the desired aim.

Rotational drive from the motor 31 is transmitted by a drive shaft 33c through a coupling 34 and to the mandrel shaft 28a. The coupling 34 serves as a linkage between shafts of differing diameters. In addition, by allowing suitable slippage at the linkage or the insertion of a safety pin, for example, it can also function as a safety device during abnormal rotation by preventing damage due to overloading of the drive unit 30 and the rotating point 28c.

Coupling of the ES servomotor 31 to the mandrel 28 through the drive shaft 33c has been described above. However, it is also possible to connect the mandrel shaft 28a directly to the drive unit 30, in which case there is no need for a drive shaft 33c and a coupling 34.

In the arrangement shown in FIG. 7, an airtight junction component (referred to hereinafter as a "rotary seal") 80 for supplying the liquid or gas core material is provided on the upstream side of the mandrel shaft 28a. The rotary seal 80 and a core material supply line 81 proper are kept from rotating by an internal sealant and a ball bearing construction. Thus, only the mandrel shaft 28a which is disposed such as to pass through the interior of the rotary seal 80 rotates.

The mandrel shaft 28a positioned at the interior of the rotary seal 80 has one or two holes for communication between the outside of the mandrel shaft 28a and the central cavity 28e therein. The size and number of holes in the mandrel shaft 28a may be freely selected within a range that does not compromise the strength of the rotating point 28c on the mandrel or the mandrel shaft 28a.

As already noted above, the portion of the mandrel shaft 28a that passes through the die body 27 is held by a shaft seal 152 which prevents resin leakage. Airtightness and heat resistance within the central cavity 28e in the portion of the mandrel shaft 28a located between the rotary seal 80 and the drive unit 30 can be ensured by packing the central cavity 28e with a suitable material such as silicon rubber or metal.

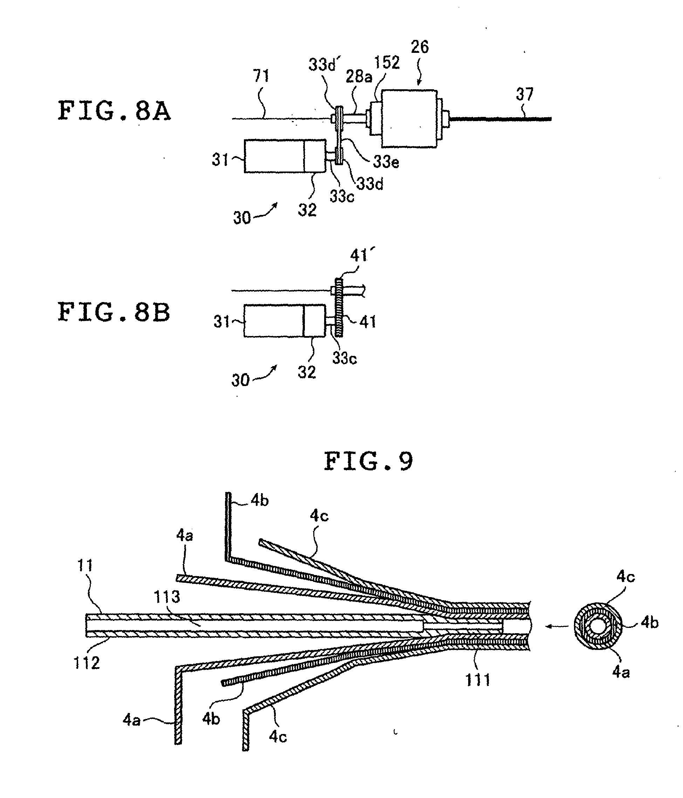

FIG. 8 shows an example of an offset coupling arrangement between the extrusion die 26 and the drive unit 30 which can be employed in cases where a solid core material such as copper wire 71 is used as the core material 70.

A copper wire 71 is fed by a core material delivery unit (not shown) and passed through the central cavity 28e within the mandrel 28 from the mandrel shaft 28a to the rotating point 28c.

Specific examples of suitable offset systems include those in which rotation of the motor 31 is transmitted to the rotating point 28c by a belt pulley (FIG. 8A) or by gears (FIG. 8B).

In the belt pulley system shown in FIG. 8A, a belt 33e transmits rotation by the drive unit 30 from a first rotary pulley 33d that is coaxial with a drive shaft 33c to a second rotary pulley 33d' that is coaxial with the mandrel shaft 28a, which in turn causes the rotating point 28c of the mandrel to turn.

In the gear system shown in FIG. 8B, rotation is transmitted to the rotating point 28c of the mandrel by a first gear 41 that is coaxial with the drive shaft 33c and a second gear 41' that meshes with the first gear 41 and is coaxial with the mandrel shaft 28a.

It should be noted that direct coupling systems and offset coupling systems suitable for use in the present context of are not limited to the configurations described above.

An example of the manufacturing process involved in the production of medical tubing 1 having the three-layer construction shown in FIG. 3 using an extrusion die 26 like that described above is described in detail below while referring to FIG. 7, which depicts a case in which the core material is air, and to FIG. 9, which schematically shows the resin stream lamination step within the extrusion die 26 and the tubing cross-section following lamination.

Air is fed at a fixed pressure from the core material supply line (air distribution line) 81 through the rotary seal 80 and into the central cavity 28e inside the rotating point 28c of the mandrel 28.

The molten resins 4a to 4c delivered from extruders 2a to 2c are respectively fed under pressure through resin inlets 30a to 30c to branched channels 31a to 31c which open up into tubular passages within the die body 27, shaped at the female die member/mandrel point resin flow-combining channel 38 to a dimension and shape close to the final target tubular body shape, and continuously extruded as medical tubing having a three-layer construction composed of an inner layer 4, an intermediate layer 2 and an outer layer 5.

Inner layer 4a in FIG. 9 incurs shear forces due to rotational friction from the rotating point 28c. If the intermediate layer 4b has, as will be discussed subsequently, high flow properties, the outer layer 4c incurs little shear force.

By giving the resin making up the filler-containing intermediate layer 4b a lower melt viscosity (meaning a higher melt flow rate (MFR), as determined according to JIS-K7210) than that of the resins making up the inner layer 4a and the outer layer 4c, and thus imparting the intermediate layer with high flow properties, the intermediate layer is made to act as a lubricating layer. That is, the intermediate layer slides in much the same way as a ball bearing and flows actively in the circumferential direction, effecting orientation of the filler. More specifically, referring to FIG. 5, after each of the resin layers have been laminated, the inner layer 4a of the resin melt in contact with the tapered section 28b of the mandrel 28 undergoes rotation. If, at this time, the intermediate layer 4b has good flow properties and slides easily, the intermediate layer 4b will incur a rotational shear force. This shear force will have substantially no influence on the outer layer 4c. Accordingly, the inner and outer layers which have a relatively high melt viscosity and are rigid do not flow, whereas the intermediate layer which has a relatively low melt viscosity and good flow properties incurs the influence of shear forces and rotates in the circumferential direction, resulting in the circumferential orientation of the filler present within intermediate layer 4b over the tubular body. The filler present in the intermediate layer 4b can be oriented at the circumferentially with respect to the tubing axis by suitable combination of such parameters as the extrusion conditions (e.g., resin extrusion rate, rotational speed of the mandrel 28) and the materials selected for use (e.g., resin viscosity).

### EXAMPLES

Examples of the invention and comparative examples are given below by way of illustration.

### Example 1 and Comparative Example 1

### Materials:

A three-layer medical tubing (referred to below as "tubular body") from the two kinds of materials described below were formed using the extrusion die shown in FIG. 5. In Example 1, the mandrel was rotated during extrusion at various speeds. In Comparative Example 1, the mandrel was not rotated (rotational speed, 0 rpm).

The outer and inner layers in these examples were made of a polyurethane resin (grade E-998 manufactured by Nippon Miractran Co., Ltd.; abbreviated below as "E-998"). The intermediate layer was made of a polyurethane resin compound containing 76 wt % of a soft magnetic metal flat powder (the compound was a sample furnished by Mate Co., Ltd., and is abbreviated below as M80PU).

The respective melt indices (MI) of the above materials were measured at 205°C and 215°C in accordance with ASTM-D570.

**Table 1**

| | | |
|---|---|---|
| (units: g/10 min) | | |

| | Melt index measurement temperature | |
|---|---|---|
| | 205°C | 215°C |
| E-998 (outer and inner layers) | 11.4 | 28.9 |
| M80PU (intermediate layer) | 296 | 623 |

### Extrusion:

The above materials were respectively fed to three extruders, the extruder and die temperatures were adjusted such as to set the polymer temperature (extrusion temperature) at 205°C, and the three melts were fed under pressure to the rotating die of the invention and extruded as a tubular body.

The resulting tubular body had an outside diameter of 2.0 mm and an inside diameter of 1.3 mm. The outside and inside diameters (expressed below as outside diameter x inside diameter) for each layer were as follows: outer layer, 2.0x1.7 mm; intermediate layer, 1.7x1.5 mm; inside layer, 1.5x1.3 mm. During extrusion, the tubular body was hauled off at a speed of 12 m/min.

The rotational speed of the rotating point on the mandrel was raised in small increments, from which it was found that the disarray in the intermediate layer observed at a speed of 0 rpm improved markedly at speeds of 50 rpm or more. At a speed of 100 rpm, a very well-defined layer structure was observed. Rotation at speeds greater than 100 rpm produced substantially no further change in appearance.

### Laminated Tubular Body:

FIG. 11A is a photograph (magnification, 50x) of the cross-section of the laminated tubular body obtained above by extrusion at a rotational speed of 100 rpm, and FIG. 11B shows an enlarged view (200x) of a portion of the same photograph. FIG. 12A is a photograph (50x) of the cross-section of the laminated tubing obtained above by extrusion at a rotational speed of 0 rpm, and FIG. 12B shows an enlarged view (200x) of a portion of the same photograph.

Schematic cross-sectional views of corresponding extruded tubular bodies are shown in FIG. 10. When the mandrel point is rotated at a sufficient speed with respect to the line speed, a uniform cross-section like that shown in FIG. 10A is obtained. However, at a rotational speed of 0 rpm, the boundaries of the intermediate layer become indistinct, as shown in FIG. 10B.

Table 2 below shows the relationship in these examples between the inside and outside diameters of the tubular body and the tubular body boundary improving effects.

**Table 2**

| Rotational speed (rpm) | Outside diameter (mm) | Inside diameter (mm) | Boundary disarray (as observed under a microscope) |
|---|---|---|---|
| 0 | 2.06 | 1.27 | large |
| 25 | 2.08 | 1.26 | moderate |
| 50 | 2.11 | 1.28 | small |
| 100 | 2.05 | 1.23 | none |
| 150 | 2.11 | 1.25 | none |
| 175 | 2.09 | 1.22 | none |
| 200 | 2.09 | 1.25 | none |

As is apparent from Table 2, the inside and outside diameters of the tubular bodies obtained in the example according to the invention remained within the range of error and thus substantially unchanged. That is, substantially no reduction in the diameter of the tubular body due to the Weissenberg effect was observed. This is probably because the intermediate layer having a low melt viscosity exhibited slip between the inner and outer layers, and thus absorbed the normal stress effect and oriented without a reduction in diameter.

The pressure resistances of the tubular bodies obtained in these examples were measured. Almost all of the tubular bodies extruded at 0 rpm in the comparative example experienced pinhole rupture at pressures of several atmospheres. By contrast, most of the samples extruded at a rotational speed of 50 rpm or more in Example 1 according to the invention did not experience pinhole rupture at pressures of several atmospheres, and were even able to withstand pressurization to 20 atmospheres or more, indicating a markedly improved pressure resistances (hoop strength). Moreover, in these latter samples, the soft magnetic flat powder was suitably oriented in the circumferential direction, enhancing the electromagnetic shielding performance.

The pressure resistance was determined by measuring the pressure at which the tubing sample ruptured when supplied with nitrogen from a pressurized cylinder.

### Example 2

The hollow tubular body having a three-layer construction shown in FIG. 3 was manufactured using the extrusion die shown in FIG. 5. A polyester elastomeric resin (grade P-280B made by Toyobo Co., Ltd.; abbreviated below as "P280"; this was a neat resin that contained no filler) was used as the resin making up the inner layer 4 and the outer layer 5. A resin compound (made to special order by Mate Co., Ltd.; abbreviated below as "TRM-PW-02") containing 50 parts by weight of P280, 25 parts by weight of EEA (PES-250, made by Nippon Unicar Co., Ltd.) as the adhesive resin, and 25 parts by weight of potassium titanate as the filler was used in the intermediate layer 2. The melt flow rates (MFR) of these resin materials were measured in accordance with JIS-K7210. The P280 resin making up the inner layer 4 and the outer layer 5 had a melt flow rate at 230°C of 17 g/10 min. The resin compound TRM-PW-02 making up the intermediate layer 2 had a melt flow rate at 230°C of 153 g/10 min.

Medical tubing having the three-layer construction shown in FIG. 3 was produced by coextrusion using the extrusion die shown in FIG. 5 and three 15 mm extruders. The extruder and die temperatures were set so as to give a resin temperature of 230°C, the mandrel rotational speed was 200 rpm, and the tubing haul-off speed was 7 m/min. Dimensions of each layer of the medical tubing thus obtained are given below as the outside diameter by inside diameter.
Overall shape (OD x ID): 2.59 mm x 2.11 mm
Outer layer: 2.59 mm x 2.43 mm
Intermediate layer: 2.43 mm x 2.27 mm
Inner layer: 2.27 mm x 2.11 mm
Examples 3 to 5, Comparative Example 2

In Examples 3 to 5 of the invention, the same procedure as described above was used to produce medical tubings having a three-layer construction, although the mandrel was rotated at a different speed in each case (50 rpm, 100 rpm, 150 rpm). In Comparative Example 2, aside from not rotating the mandrel, medical tubing having a three-layer construction was produced by the same procedure as described above. Table 3 shows, along with the results obtained in Example 2 of the invention, the outside and inside diameters of the medical tubings obtained in Examples 3 to 5 and in Comparative Example 2. The outside and inside diameters shown in this table are those of the medical tubing itself, not of the individual layers.

**Table 3**

| Mandrel rotational speed (rpm) | Outside diameter (mm) | Inside diameter (mm) |
|---|---|---|
| 0 (Comparative Example 2) | 2.61 | 2.20 |
| 50 (Example 3) | 2.66 | 2.25 |
| 100 (Example 4) | 2.59 | 2.18 |
| 150 (Example 5) | 2.60 | 2.15 |
| 200 (Example 2) | 2.59 | 2.11 |

| | | |
|---|---|---|
| Make-up of resin layers: P280/TRM-PW-02/P280 Extrusion temperature: 230°C Tubing haul-off speed: 7 m/min | | |

As is apparent from Table 3, regardless of the mandrel speed of rotation, the inside and outside diameters of the medical tubings produced were about the same, and fell within the range of error (±0.1 mm). Substantially no reduction in the diameter of the medical tubing due to a normal stress effect was observed. This is most likely because the intermediate layer, which has a relatively low melt viscosity (high MFR), experienced slippage between the inner layer and the outer layer, absorbed the normal stress effect, and oriented without a reduction in diameter.

To ascertain the influence of mandrel rotation on the boundary state of the intermediate layer in the medical tubing, cross-sectional micrographs were taken of the medical tubing produced in Example 2 (mandrel rotational speed, 200 rpm) and the medical tubing produced in Comparative Example 2 (mandrel rotational speed, 0 rpm). To ascertain the influence of mandrel rotation on filler orientation, portions of these cross-sectional images of the medical tubings obtained in Example 2 and Comparative Example 2 were enlarged under a scanning electron microscope (SEM) and photographed. FIG. 13A, which is a scanning electron micrograph (magnification, 1,000x) showing an enlarged portion of a cross-sectional image of the medical tubing obtained in Example 2, shows that the filler is oriented circumferentially. FIG. 13B, which is a cross-sectional micrograph (magnification, 50x) of the medical tubing obtained in Example 2, shows that the intermediate layer of the medical tubing is orderly and the boundary state is well-defined. FIG. 14A, which is a scanning electron micrograph (magnification, 1,000x) of an enlarged portion of a cross-sectional image of the medical tubing obtained in Comparative Example 2, shows that the filler is randomly distributed rather than being oriented in a specific direction. FIG. 14B, which is a cross-sectional micrograph (magnification, 50x) of the medical tubing obtained in Comparative Example 2, shows that the boundaries of the intermediate layer in the medical tubing are less well defined.

The three-layer medical tubings produced in Examples 2 to 5 and in Comparative Example 2 were subjected to a three-point bending test to evaluate their physical properties, in addition to which their resistances to kink were evaluated.

### Three-Point Bending Test:

The medical tubings of Examples 2 to 5 and of Comparative Example 2 were supported between two points spaced 13 mm apart, a crosshead was pushed against the center portion of the tubing, and the applied load at the time of 1 mm deflection was measured. The three-point bending test was carried out on five medical tubing specimens from each example, and the average of the five results was determined. FIG. 15 is a bar graph showing the relationship between the rotational speed of the mandrel (rpm) during production and the three-point bending load (gf). Each load value shown in FIG. 15 is the average of five measurements. It is apparent from FIG. 15 that the three-point bending load rises with increasing mandrel rotational speed, thus confirming a rise in the strength of the medical tubing perpendicular to the tubing axis.

### Loop Kink Test:

The kink resistance of the medical tubings obtained in Examples 2 to 5 and in Comparative Example 2 were evaluated by carrying out a loop kink test. A 300 mm length of medical tubing produced as described above was passed through a plate bearing two 2.7 mm diameter holes separated by an interval of 30 mm, formed into a loop, and both ends of the tubing were pulled away from the plate. The loop height ("kink point") just prior to kink formation was determined. The results of the loop kink test are given in FIG. 16, which is a graph showing the relationship between the rotational speed of the mandrel (rpm) during production and the kink point. It is apparent from FIG. 16 that the kink point becomes lower with increasing mandrel rotational speed, thus confirming a rise in the kink resistance of the medical tubing.

### Production and Evaluation of Introducer Sheaths:

Three-layer sheaths having an outside diameter of 2.6 mm, an inside diameter of 2.2 mm and a length of 100 mm were produced in the same way as in Example 2. This was Example 6 according to the invention. The resin materials used in the respective layers (inner layer, intermediate layer, outer layer) and the production conditions were the same as in Example 2.

The resulting sheath was heat-treated near the distal end to form a tapered section (length, 2.8 mm; minimum outside diameter, 2.2 mm).

The sheath in which a tapered section had thus been formed was attached to a hub equipped with a sheath support, a valve and a branch tube, thereby giving the intrbducer sheath shown in FIG. 4.

Specimens of the sheath produced in Example 6 were subjected to a bend kink test to evaluate the kink resistance without first imparting a taper to the sheath and attaching it to a hub. A catheter-like metal rod of circular cross-section and having an outside diameter substantially equivalent to the inside diameter of the sheath was inserted into the sheath lumen, and the tubing was pushed downward by hand at a point 50 mm in front of the inserted tip of the metal rod. The angle to the horizontal of the bent section of the sheath when kinking occurred was measured.

Bend kink tests were also performed on sheaths produced, as in Comparative Example 2, without mandrel rotation (Comparative Example 3); on sheaths produced in the same way as in Example 6 and in Comparative Example 3, aside from the absence of filler in the intermediate layer (Comparative Example 4 and Comparative Example 5); and on sheaths produced according to the prior art, specifically sheaths which are currently available commercially (manufactured by Terumo Corporation under the trade name Radifocus Introducer IIH; Comparative Example 6). The prior-art sheaths have a single-layer construction. The resin making up the layer is a fluoroplastic (ETFE), the inside diameter is 2.12 mm, and the outside diameter is 2.62 mm.

The results of the bend kink test are given in FIG. 17. It is apparent from FIG. 17 that the sheath in Example 6, in which the intermediate layer contained 25 wt % of filler and the rotational speed of the mandrel was 200 rpm, had a bending angle from 1.24 to 1.35 times larger than the sheath in Comparative Example 3 which was produced without mandrel rotation, and the sheaths in Comparative Examples 4 and 5 which were produced with an intermediate layer containing no filler. Hence, the sheath in Example 6 demonstrated an improved kink resistance. This sheath also had a bending angle 1.55 times larger than the Comparative Example 6 sheath produced according to the prior art, again demonstrating improved kink resistance.

As described in detail above and demonstrated in the foregoing examples, the medical tubing of this invention has a filler-containing resin layer in which the filler is oriented at an angle of more than ±0 degrees and up to 90 degrees to the tubing axis. This resin layer provides the tubing with improved maneuverability, including pushability, ability to transmit torque, trackability and kink resistance. By giving the medical tubing of the invention a laminated construction in which a filler-free resin layer is provided on at least the inside and outside of the filler-containing resin layer, the filler-containing resin layer is covered with the filler-free resin layer or layers, preventing the filler from being exposed on the tubing surface and thus enhancing safety. Moreover, an excellent interlayer adhesion can be achieved in such laminated medical tubing by having the filler-containing resin layer made of the same type of resin as that used in the filler-free resin layers provided on the outside or inside thereof, and by also including an adhesive resin which bonds with the principal resin in the layers.

Introducer sheaths in which the inventive medical tubing serves as the sheath portion have a kink resistance which is dramatically improved over that of prior-art introducer sheaths.

The medical tubing of the invention lends itself well to use not only in introducer sheaths, but also in other medical instruments that are required to be thin-walled and endowed with good kink resistance and pressure resistance, including various catheters such as PTCA catheters and microcatheters.

Although some preferred embodiments have been described, many modifications and variations may be made thereto in light of the above teachings.

## Claims

1. A medical tubing comprising an elongated hollow tubular body which is open at both ends and extends continuously therebetween, **characterized in that** the hollow tubular body has a three-layer construction which is composed of an outside and an inside filler-free resin layers (4, 5) and an intermediate filler-containing resin layer (2), the filler (3) being oriented within the filler-containing resin layer in a circumferential direction with respect to the hollow tubular body.

2. The medical tubing of claim 1, wherein at least 30 % of the filler in the resin layer is oriented in the same direction.

3. The medical tubing of claim 1, wherein the filer-containing resin layer (2) is composed of a principal resin, an adhesive resin and a filler.

4. The medical tubing of claim 1, wherein the filler (3) is composed of whiskers.

## Patentansprüche

1. Ein medizinischer Schlauch mit einem gestreckten, hohlen, schlauchartigen Hauptteil, das an beiden Enden offen ist und das sich dazwischen durchgehend erstreckt, **dadurch gekennzeichnet, dass** das hohle, schlauchartige Hauptteil eine Ausführung mit drei Schichten aufweist, welche Ausführung aus einer äußeren und einer inneren, von einem Harzträger bzw. Füller freien Harzschicht (4, 5) und einer zwischenliegenden, einen Harzträger bzw. Füller enthaltenden Harzschicht (2) zusammengesetzt ist, wobei der Füller (3) innerhalb der den Füller enthaltenden Harzschicht in einer Umfangsrichtung in Bezug auf das hohle, schlauchartige Hauptteil orientiert ist.

2. Der medizinische Schlauch nach Anspruch 1, wobei mindestens 30% des Füllers in der Harzschicht in der gleichen Richtung orientiert ist.

3. Der medizinische Schlauch nach Anspruch 1, wobei die den Füller enthaltende Harzschicht (2) von einem Hauptharz, einem Bindeharz und einem Füller gebildet wird.

4. Der medizinische Schlauch nach Anspruch 1, wobei der Füller (3) aus Nadelkristallen gebildet ist.

## Revendications

1. Tubulure médicale comprenant un corps tubulaire creux allongé qui est ouvert aux deux extrémités et s'étend de façon continue entre elles, **caractérisé en ce que** le corps tubulaire creux a une structure à trois couches qui se compose de couches de résine sans charge extérieure et intérieure (4, 5) et d'une couche de résine intermédiaire contenant une charge (2), la charge (3) étant orientée, à l'intérieur de la couche de résine contenant une charge, dans une direction circonférentielle par rapport au corps tubulaire creux.

2. Tubulure médicale selon la revendication 1, dans laquelle au moins 30 % de la charge présente dans la couche de résine sont orientés dans la même direction.

3. Tubulure médicale selon la revendication 1, dans laquelle la couche de résine contenant une charge (2) se compose d'une résine principale, d'une résine adhésive et d'une charge.

4. Tubulure médicale selon la revendication 1, dans laquelle la charge (3) est constituée de trichite.
